Europäisches Patentamt

European Patent Office

Office européen des brevets

(11) Publication number: **0 321 145**
**A2**

(12) **EUROPEAN PATENT APPLICATION**

(21) Application number: **88311621.2**

(22) Date of filing: **08.12.88**

(51) Int. Cl.4: **G01N 33/543 , G01N 33/52 , B01L 3/00 , //G01N33/558**

(30) Priority: **15.12.87 US 133339**

(43) Date of publication of application:
**21.06.89 Bulletin 89/25**

(84) Designated Contracting States:
**DE FR IT NL**

(71) Applicant: **PALL CORPORATION**
**30 Sea Cliff Avenue**
**Glen Cove New York 11542(US)**

(72) Inventor: **Pascale, Frank Richard**
**12 Francis Court**
**Glen Cove New York 11542(US)**
Inventor: **Latham, Malcolm James**
**43 Cutlers Lane**
**Stubbington Fareham Hants. PO 14 2JT(GB)**
Inventor: **Matkovich, Vlado Ivan**
**11 Old Estate Road**
**Glen Cove New York 11542(US)**

(74) Representative: **Corin, Christopher John et al**
**Mathisen Macara & Co. The Coach House 6-8**
**Swakeleys Road**
**Ickenham Uxbridge UB10 8BZ(GB)**

(54) **Immunodiagnostic device.**

(57) A flow enhanced immunodiagnostic device is provided comprising a hollow elongate member having first and second openings and a porous liquophilic membrane positioned across the first opening. The porous liquophilic membrane may also contain a test reagent or an activating agent capable of reacting with a test reagent which itself is capable of reacting with a predetermined analyte and producing an indicia of the presence of the analyte, the second opening being adapted to be operatively associated with means for generating a pressure differential between the interior of the elongate member and the exterior thereof, whereby a liquid test sample containing an analyte may be introduced into the interior of the elongate member by passing through the first opening and the porous hydrophilic membrane and any insoluble substances present in the liquid test sample accumulates on a surface of the porous liquophilic membrane.

FIG. I

## IMMUNODIAGNOSTIC DEVICE

The present invention is directed to a flow enhanced diagnostic device. More particularly, the present invention is related to a diagnostic device which is capable of providing accurate test information without interference from insoluble solid matter.

Diagnostic tests employing dipsticks are not new and have been employed for many years both in home test kits and for tests performed in the laboratory by skilled technicians. Such dipsticks are convenient to use, disposable, frequently inexpensive, and are in some instances capable of providing semi-quantitative results, although they are more commonly employed in qualitative tests. Generally, the dip sticks are designed for easy manual handling and are typically narrow, long, semi-rigid plastic or cardboard strips on which one or more diagnostic reagents are placed. Many dipsticks produced in recent years have been provided with a pad of porous membrane affixed to one end by adhesives, double-sided tape, or edge welding.

In performing diagnostic tests with membrane pad-affixed dipsticks, the user is generally supplied with a dipstick which includes at its active end a membrane pad which has previously been treated with a diagnostic test reagent. The user brings the active end of the dipstick in contact with the liquid being tested. Such liquids may include, among others, urine specimens, mouth or vaginal swab samples, suspended feces samples, plasma, or serum samples. The dipstick is allowed to remain in contact with the liquid being tested for the amount of time prescribed for the particular test being performed. During this time, the liquid must diffuse through the exposed face of the membrane where it reacts with the test reagent, typically a biologically active material such as a bio specific molecule, which is bound to the membrane. At the end of this time, the dipstick is removed from the sample liquid and is typically treated with a series of other reagents to develop an indicia of the presence of the analyte being tested for, typically manifested by a color change. Some of the treatments may, however, include wash steps, substrate addition, and conjugate addition. The net result is usually a color-producing reaction, if the liquid under test originally contained the biomolecule the dipstick was designed to detect.

Although convenient and offering improved results in comparison to standard dipsticks which lacks membrane pad, these membrane pad-affixed dipsticks suffer from several disadvantages. The first relates to the types of sample which may be used with such dipsticks. At one extreme and providing no difficulties are pure liquids which are typically free from extraneous material, as for example, samples of serum or plasma. The other extreme includes samples in which insoluble solid or particulate matter is present, typically suspended in a liquid. Examples of such heterogeneous mixtures include swab samples or suspended fecal samples, which contain materials such as mucus or solid particles. The suspended solids or gel-like materials tend to coat and block the membrane surface. This results in inhibition of free flow of liquid into the membrane and the insoluble matter may also obscure any color change which may have occurred on the surface of the membrane. Such shortcomings can frequently cause anomalous test results. Thus, use of dipsticks in many tests may be limited to liquids which are reasonably free from particulate debris or may require that a separation technique be applied to the liquid sample to remove such insoluble matter prior to testing the liquid.

The second limitation on the use of dipsticks relates to the manner in which various liquids, such as the sample, wash solution, and reagents, pass into and out of the membrane. In a conventional membrane pad-affixed dipstick, one face of the membrane, in use, is exposed to liquids and the other is affixed to a non-porous backing which forms the support or dipstick body itself. Thus liquids which pass through the exposed membrane surface into the membrane pad can do so only be diffusion processes. The maximum rate for diffusion is limited by the initial concentration gradient between the bulk liquid surrounding the dipstick and the liquid in the membrane itself. With time, the concentration gradient falls and diffusion into or out of the membrane slows down. Washing steps, even when performed under running water, are still limited by diffusion. Such diffusion limitations usually result in increased test times, along with the risk of false results it the duration of wash steps is not followed carefully. Some dipsticks are available in which the membrane is affixed so that both sides of the membrane pad are exposed to liquid. However, diffusion is still limited in such designs although enhanced compared to a single surface membrane type of a dipstick.

Accordingly, the subject invention provides for a flow enchanced diagnostic device which functions in many respects as a dipstick in the manner in which tests are performed and interpreted. The subject invention provides for a flow enhanced immunodiagnostic device comprising a hollow elongate member having first and second openings;

and a porous liquophilic membrane positioned across said first opening, said porous liquophilic membrane containing an activating agent capable of reacting with a test reagent, said second opening adapted to be operatively associated with means for generating a pressure differential between the interior of said elongate member and the exterior thereof, whereby a liquid test sample may be introduced to the interior of said elongate member bypassing through said porous liquophilic membrane and any insoluble substances present in said liquid test sample accumulates on a surface of said porous liquophilic membrane.

The subject invention further provides for a flow enhanced immunodiagnostic device comprising a hollow elongate member having at least first and second openings; a porous liquophilic membrane positioned across said first opening and containing an activating agent capable of reacting with a test reagent; and means associated with said second opening for permitting ingress of liquid into said elongate member through said second opening while retarding egress of liquid from said elongate member through said second opening, whereby a liquid test sample maybe introduced to the interior of said elongate member through said ingress means and liquid is expelled from said reservoir exclusively through said porous liquophilic membrane while any insoluble substance present in said liquid test sample accumulates on an inner surface of said porous liquophilic membrane.

The subject invention further provides for a flow enhanced immunodiagnostic device comprising a hollow elongate member having first and second openings; and a porous liquophilic membrane positioned across said first opening, said second opening adapted to be operatively associated with means for generating a pressure differential between the interior of said elongate member and the exterior thereof, whereby a liquid test sample may be introduced into the interior of said elongate member bypassing through said porous hydrophilic membrane and any insoluble substances present in said liquid test sample accumulates on a surface of said porous liquophilic membrane.

The subject invention further provides for a flow enhanced immunodiagnostic device comprising a hollow elongate member having at least first and second openings; a porous liquophilic membrane positioned across said first opening; and means associated with said second opening for permitting ingress of liquid into said elongate member through said second opening while retarding egress of liquid from said elongate member through said second opening, whereby a liquid test sample maybe introduced to the interior of said elongate member through said ingress means and liquid is expelled from said reservoir exclusively through said porous liquophilic membrane while any insoluble substance present in said liquid test sample accumulates on an inner surface of said porous liquophilic membrane.

The subject invention further provides for a flow enhanced immunodiagnostic device comprising a hollow elongate member having first and second openings; and a porous liquophilic membrane positioned across said first opening, said porous liquophilic membrane containing a test reagent capable of reacting with an analyte and producing an indicia of the presence of the analyte, said second opening adapted to be operatively associated with means for generating a pressure differential between the interior of said elongate member and the exterior thereof, whereby a liquid test sample containing a substantially soluble analyte may be introduced into the interior of said elongate member by passing through said first opening and said porous liquophilic membrane and any insoluble substances present in said liquid test sample accumulates on a surface of said porous liquophilic membrane.

The subject invention further provides for a flow enhanced immunodiagnostic device comprising a hollow elongate member having at least first and second openings; a porous liquophilic membrane containing a test reagent capable of reacting with a predetermined analyte and producing an indicia of the presence of the analyte, said liquophilic membrane positioned across said first opening; and means associated with said second opening for permitting ingress of liquid into said elongate member through said second opening while retarding egress of liquid from said elongate member through said second opening, whereby a liquid test sample containing an analyte may be introduced to the interior of said elongate member through said ingress means and liquid is expelled from said elongate member exclusively through said porous liquophilic membrane and said first opening while any insoluble substance present in said liquid test sample accumulates on an inner surface of said porous liquophilic membrane.

At some point prior to use, the membrane is treated with a test reagent and typically with an activating agent prior to treatment with the test reagent. The test reagent is suitable for providing an indicia of the presence of a specified analyte being sought in a test sample.

The inclusion of a means for creating a differential pressure overcomes the diffusion problems associated with most dipsticks and allows tests to be performed much more rapidly than with conventional types of dipsticks. The device permits a liquid sample to be introduced into the interior of the elongate member and any insoluble solid material present to be deposited on a surface of the membrane. In a preferred embodiment, the means for creating a differential pressure also allows any insoluble solid substances, such as particulate or gel-like material, accumulating on the outer surface of the membrane to be removed easily, simply by expelling the last portion of liquid sample from the reservoir through the membrane. This avoids problems associated with such particulate or gel-like material obscuring tests by coating the viewing surface of the membrane. In another preferred embodiment, the sample is introduced to the interior of the hollow elongate member through a check valve located in an additional opening in the elongate member. Purified liquid is expelled through the membrane with any insoluble solid matter accumulating on a surface of the membrane facing the interior of the hollow elongate member. Because relatively high positive pressures may be generated in this embodiment, liquid flow through the membrane is rapid and any color change on the membrane surface may be easily viewed.

Figure 1 is a perspective view of an embodiment of the flow enhanced immunodiagnostic device employing an elastomeric bulb as a means for obtaining a differential pressure.

Figure 2 is a perspective view of another embodiment of the flow enhanced immunodiagnostic device of the invention using a plunger to generate a differential pressure.

Figure 3 is a perspective view of another embodiment of the flow enhanced immunodiagnostic device incorporating a one-way valve.

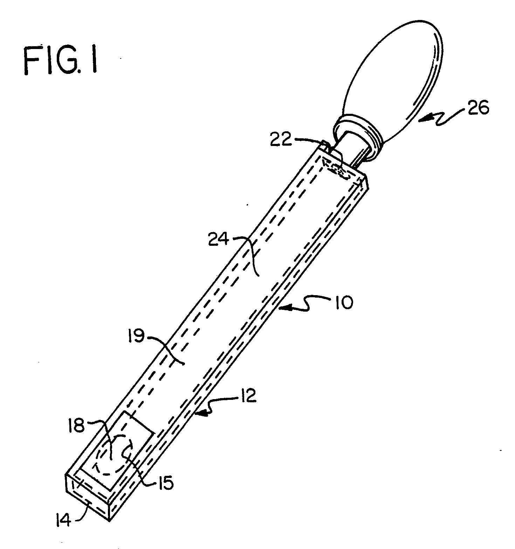

An embodiment of the flow enhanced immunodiagnostic device is illustrated in Figure 1. The flow-enhanced diagnostic device, generally designated 10, includes a hollow member 12, typically having an elongated shape and having a first or lower end 14 with a first or lower opening 15 (located at or proximate the lower end 14) and an upper end 16. The hollow member 12 preferably has a square or rectangular outer-configuration which lends itself to high volume manufacturing techniques (as discussed below). The tubular portion or passage, i.e., the inner wall of the hollow elongate member, is preferably circular. However, any other convenient shapes conducive to molding and assembly may be employed. For example, both the outer and inner configurations may be circular or rectangular.

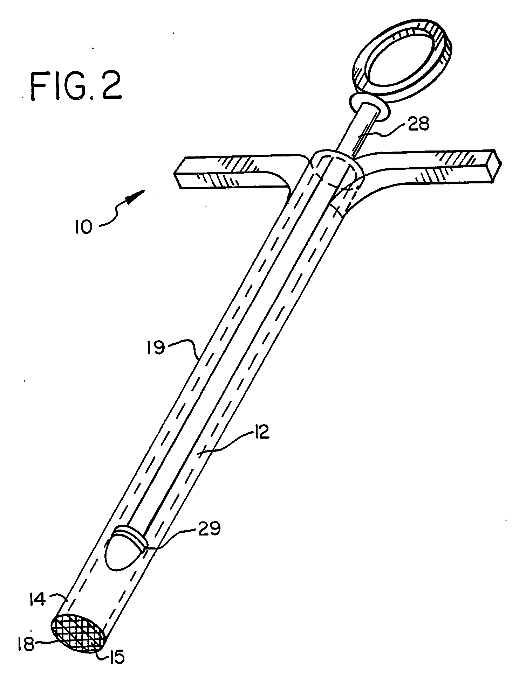

In the embodiment shown in Figure 1, the lower opening 15, having a porous structure 18 lying over or within the opening, is located in one of the sidewalls 19 proximate to the lower end 14 of the device Alternatively, the opening 15 may be located in or at the bottom wall formed at the lower end 14, as shown in Figure 2. In such an embodiment, the dimensions of the opening 15 may be such as to eliminate entirely the bottom wall; that is, the side walls 19 define the lower opening 15. Depending upon the application for which the device is employed, a large or small opening 15 may be provided. Thus, if it is desirable to concentrate the material being tested for (analyte), a smaller opening and concomitant area of gross surface of the membrane or porous structure 18 is used. However, if the test liquid contains a gel-like substance or material which readily agglomerates and tends to cling tenaciously to the surface of the membrane in a manner to reduce the flow rate, a larger opening accommodating a larger area of membrane may be employed to allow a higher flow rate. As employed herein and unless indicated otherwise, the term "surface" or "surface area" refers not only to the gross surface(s) of the structure but also, in those cases where a microporous structure such as a membrane is under consideration, to the surfaces of the micropores, i.e., the interior surfaces of the structure which define the pores and which are contacted by fluid during use.

When a device is used in which the side walls of the elongate hollow member define the opening 15 in the bottom of the device, at least a portion of the side walls may be configured so as to taper toward the opening when a smaller opening and reduced area of the membrane is desired. In such an embodiment, the lower tip of the device has the shape of a frustum, being trapezoidal in shape when the outer configuration of the device is rectangular and being conical when the outer configuration of the device is circular.

The elongate member is preferably formed from an inert, transparent, or translucent material, such as glass or plastic. Thermoplastic materials which are compatible with the biological solutions with which the flow enhanced immunodiagnostic devices are expected to have most widespread applicability are preferred. Examples of such materials are polystyrene, polycarbonates, and polyolefins, such as polypropylene and polyethylene. The material employed to make the elongate member should be substantially inert to reagents employed. The term "inert," as used herein, refers to a lack of chemical reactivity towards solvents and reagents which may be employed and substances present in the specimen, particularly in biological samples, such as bodily fluids, and at various pH conditions, particularly those encountered in biological fluids, such as body fluids.

Located at the first or lower opening 14 of the hollow elongate member 12 is a porous liquophilic and preferably hydrophilic structure, such as a membrane, 18 which is treated with a test reagent and/or an activating agent either before or after application to the hollow member 12. The structure is also preferably microporous.

As used herein, "analyte" refers to any substance or substances being analyzed for or determined by the diagnostic test. The terms "reagent", "test reagent", and like terms refer both to substances reacting directly with an analyte as well as to substances used to convert either another substance or the analyte to a substance suitable for providing an indication, typically an optical indication, of the presence of the analyte. The term includes simple chemical substances, including elements and compounds, of an ionic or molecular nature as well as more complex or macromolecular structures, such as proteinaceous materials, and includes substances of a biological nature such as antigens, antibodies, enzymes, antibody-enzyme conjugates, haptens, receptors, lectins, gene probes, and the like, as well as viruses and bacteria. The analyte may also be one or more of the substances falling within the definition of a reagent. In the case of antibodies, reagents and analytes may each be monoclonal or polyclonal antibodies. Additionally, the analyte may include drugs, peptides, cells, and organelles. A reagent may additionally include buffers, indicator molecules, and substrates.

The material from which this porous structure or membrane is formed must not react adversely with substances found in either the samples, reagents or solvents employed in the analyses or separation.

Depending upon the type of sample with which the diagnostic device is to be used, the liquophilic membrane 18 may be porous or microporous, preferably the latter. The membrane typically has an absolute pore rating suitably ranging from 0.04 to 40 micrometers. Preferably, the pore rating is 0.1 to 5 micrometers, and most preferably 0.2 to 3 micrometers. The membrane, preferably, is also skinless. Materials which are suitable for use as the membrane also have voids volumes in the range of 60 to 90 percent, preferably in the range of 75 to 90 percent. Preferred materials are hydrophilic in nature and are, therefore, easily water-wettable and tend to freely pass aqueous solutions. Examples of liquophilic materials which may be used in the present invention include, but are not limited to, polyamides, such as nylon 66 (polyhexamethylene adipamide), polyvinylidene difluoride, cellulose esters, and nitrocellulose.

Liquophilicity, as used herein, refers to the wettability of the membrane by the liquid(s) with which it is contacted. The wettability or liquophilicity of a solid structure, e.g., a membrane, is a function of that structure's critical surface energy and the surface tension of the applied liquid. If the critical surface energy is at least as high as the surface tension of the liquid, the liquid will spontaneously wet the solid structure. For example, a microporous membrane having a critical surface energy of 72 dynes/cm or higher will be wetted by water which has a surface tension of 72 dynes/cm, i.e., it is hydrophilic.

The capability of a porous structure (membrane) to be wetted by a liquid can be determined by placing a drop of liquid on the porous structure. The angle of contact provides a quantitative measure of wetting. A very high angle of contact indicates poor wetting, while a zero angle of contact defines complete or perfect wetting. Materials used as the wettable or liquophilic porous layer are characterized by being readily or spontaneously wetted by the applied liquid and have a low angle of contact with the applied liquid. Indeed, when a drop of a test liquid(s) is placed on a spontaneously wettable or liquophilic microporous membrane layer, the drop of liquid penetrates the layer and wets the membrane, effectively providing a zero angle of contact therewith.

Wettability or liquophilicity is a requisite of the materials used for the porous membranes. It is particularly preferred that such materials be capable of being spontaneously wetted. Some of the materials which are suitable or preferred for use as the porous structure or membrane are intrinsically hydrophilic or water-wettable. Others may be modified to render them hydrophilic. BIODYNE$^R$, a material preferred, is an $N_{66}$ polyamide, microporous membrane commercially available from Pall Corporation which is inherently water-wettable by virtue of its method of manufacture as evidenced in U.S. Patent 4,340,479. This patent describes a process for preparing skinless hydrophilic alcohol-insoluble polyamide membranes that are readily wetted by water. The process comprises first preparing a solution of an alcohol-insoluble polyamide resin. Induced nucleation of the solution is then achieved by addition to the solution of a nonsolvent for the polyamide resin, under controlled conditions of concentration, temperature, addition rate, and degree of agitation to obtain a visible precipitate of polyamide resin particles, thereby forming a casting solution. The casting solution is subsequently spread on a substrate to form a thin film on the substrate. The film of the casting solution is then contacted and diluted with a mixture of the solvent and nonsolvent liquids, thereby precipitating polyamide resin from the casting solution in the form of a thin, skinless, hydrophilic membrane. Finally, resulting membrane is washed to remove solvent and subsequently dried.

Polyvinylidene fluoride membranes are not inherently water-wettable but can be rendered such by an appropriate surface treatment. Microporous, poly-vinylidene fluoride membranes which have been treated to render them hydrophilic are commercially available.

Materials which are preferred for the porous structure 18 also have large surface areas. This feature permits a greater amount or higher concentration of reactant to be immobilized in the reaction layer. Accordingly, higher sensitivities may be achieved using the subject diagnostic test device.

Also included among the preferred polyamide membranes are hydrophilic, microporous, skinless polyamide membranes with controlled surface properties of the type described in U.S. Patent 4,707,266 and in U.S. Patent 4,702,840. These hydrophilic, microporous, substantially alcohol-insoluble polyamide membranes with controlled surface properties are formed by cocasting, in a manner similar to that described above, an alcohol-insoluble polyamide resin with a water-soluble, membrane-surface-modifying polymer having functional polar groups. Several of the materials having these desirable properties, which are disclosed in the two U.S. patents described above, are available from Pall Corporation under the marks CARBOXYDYNE[R] (membranes which have functional polar groups selected from the group consisting of hydroxyl, carboxyl, and amino groups, or a non-reacting combination thereof) and POSIDYNE[R] - (membranes which have surface characteristics of cationic, quaternary ammonium groups). Like the preferred hydrophilic, microporous nylon membranes which do not have controlled surface-modified polar groups present, the polyamide membranes having controlled surface properties are also, preferably, skinless; that is, they are characterized by through pores extending from surface-to-surface which are of substantially uniform size and shape. If desired, however, materials having tapered through pores, i.e., pores which are larger at one surface of the sheet, narrowing as they approach the opposite surface of the sheet, may be used.

The surface-modifying polymers used to prepare the polyamide membranes with controlled surface properties comprise polymers which contain substantial proportions of chemically functional groups, such as hydroxyl, carboxyl, amine, and imine groups. As a result, the membranes include, at their surfaces, high concentrations of functional groups such as hydroxyl, carboxyl, imine, or a combination of any of the above groups which do not react with one another. These polyamide membranes having controlled surface properties have higher concentrations of carboxyl or imine groups at their surfaces than the preferred microporous, hydrophilic, skinless polyamide membranes described above which do not have controlled surface properties, i.e., those which are formed from the preferred polyamide resin but are not cocast with a surface-modifying polymer.

As suggested above, suitable materials should also be capable of being treated with and retaining or immobilizing a test reagent, and/or an analyte. The test reagent, which may be of ionic, molecular, or macromolecular nature may be immobilized on the porous structure or membrane by strong physical forces or by being bonded in some manner, such as covalent chemical coupling, preferably by a covalent bond, to the surface of the microporous, liquophilic reaction membrane layer.

The porous structure or membrane 18 may be treated by any method known to one of skill in the art to deposit and/or bind reagents thereto. As indicated above, the reagent may be of an ionic, molecular, or macromolecular nature. As a diagnostic tool to provide a visible change, the reagent may be one or a combination of substances which are initially colorless or of a particular color and which, upon reaction with an analyte, provides a visible indicia or an optically measurable response. Other possible means for detecting a positive test include the use of suitable labels, such as the formation between the immobilized test reagent and the analyte of a complex or compound which is appropriately labeled by any known technique, such as enzymatic substrate labels, radioactive labels, or the like.

Although treatment of the porous structure with a suitable reagent(s) may be performed at the time at which diagnostic tests are to be performed, including addition of the test reagent(s) both immediately preceding and following contact of the sample containing the analyte with the membrane 18, the subject invention is expected to have greatest application to, and a preferred embodiment includes, a membrane in which the membrane 18 has been pretreated with at least one test reagent and/or an activating agent. Typically, pretreatment is conducted after the membrane has been sealed across the opening 14 but before the device is shipped to a user. If the reagent(s) is not heat sensitive, the membrane may be treated before assembling the composite membrane on the elongate member 12.

The test reagent may be immobilized on the surface of the membrane by any method which assures that the reagent will not be removed by being dissolved or leached from the membrane with a liquid, such as that found in a solution of another reagent applied to the membrane or the specimen sample itself. Thus, physical or chemical bonding of the test reagent to the membrane may be used but the latter is preferred, particularly in the form of covalent bonds.

A useful method of binding reagents of a molecular nature, especially macromolecules, and particularly those of a biological nature, is disclosed in U.S. Patent 4,693,985. This patent describes a method for immobilizing a wide range of biologically active substances as acceptor molecules (test reagents) on active membranes (hydrophilic, microporous skinless, polyamide membranes). The acceptor molecule-bound membranes described in the application are capable of immobilizing and binding a wide variety of biologically-active compounds, specifically ligands, to the acceptor molecules. Using such membranes permits the testing of bodily fluids, such as blood, serum, plasma, urine, saliva, and the like, and testing for particular substances by chemical assays or immunoassays, such as those where a specific label is employed, such as one indicating enzyme activity, an electromagnetic energy absorbing and/or emitting label, such as a fluorescent label, or a radioactive label. The macromolecules which are used as reagents and bound to the reaction layer or which are determined by using the flow enhanced immunodiagnostic device generally include materials of a biological nature and are frequently proteinaceous in nature. The reagent or acceptor molecule bound directly to the reaction layer or the ligand being tested for includes such substances as immunoglobulins or antibodies, either polyclonal or monoclonal, antigenic substances, apoproteins, receptors, glycoproteins,lectins, carbohydrates, hormones, enzymes, carrier proteins, heparin, coagulation factors, enzyme substrates, inhibitors, cofactors, nucleic acids, etc.

An embodiment which is particularly preferred is a chemically active membrane 18. This membrane includes an activating agent immobilized on the surface of the membrane. In most instances, the activating agent is chemically bound, preferably covalently bound, to the membrane surface such that a residue of the activating agent is the actual species bound to the membrane, which residue has a functional group capable of reacting with a test reagent. Thus, the original activating agent, most typically, is a compound having at least two functional groups, one of which is capable of reacting with moieties present on the surface of the membrane and another functional group capable of reacting with a moiety in a test reagent (acceptor molecule). Once the activating agent has reacted with both the membrane and the test reagent, i.e. , the chemically active membrane has reacted with a test reagent, the activating agent functions as a "linking" agent to link the membrane to the test reagent. Since the test reagent or acceptor molecule is frequently a biologically active substance, the membrane which has a test reagent immobilized on its surface may be termed a "biologically active" membrane, or a similar term, even though the test reagent may not be of a biological nature, and those membranes having only an activating agent immobilized thereon are designated an "activated" membrane, "chemically activated" membrane or by a similar term.

A preferred material is a chemically active membrane prepared as described in U.S. Patent 4,693,985. This material is a surface-modified polyamide having a residue of trichloro-s-triazine bound thereto as an activating agent capable of covalently bonding to a test re-agent. Such material is available from Pall Corporation as IMMUNODYNE™.

The membrane 18 may be secured to the hollow member 12 at the first opening 15 by conventional methods, such as heat welding, ultrasonic welding, or adhesive means. Preferably, the membrane is hermetically sealed to the hollow member, thereby requiring all liquids which pass into a reservoir 24 defined by the internal walls of the hollow member and the porous hydrophilic membrane to pass exclusively through the membrane.

In the embodiment illustrated in Figure 1, attached to the hollow member 12 at the second or upper opening 22 is a means for effecting a differential pressure between the inside and outside of the hollow member 12. The differential pressure means may be an aspirator bulb, such as the illustrated simple elastomeric bulb 26, a piston, or the like.

In the device shown in Figure 2, the means for effecting a differential pressure between the inside and outside of the hollow member 12 takes the form of a piston or plunger 28. The piston has cross-sectional dimensions and configuration which are commensurate with the shape and internal dimensions of the hollow member 12 such that the piston slides tightly within the hollow member 12 and in so doing creates a pressure differential corresponding to a pressure above or below atmospheric.

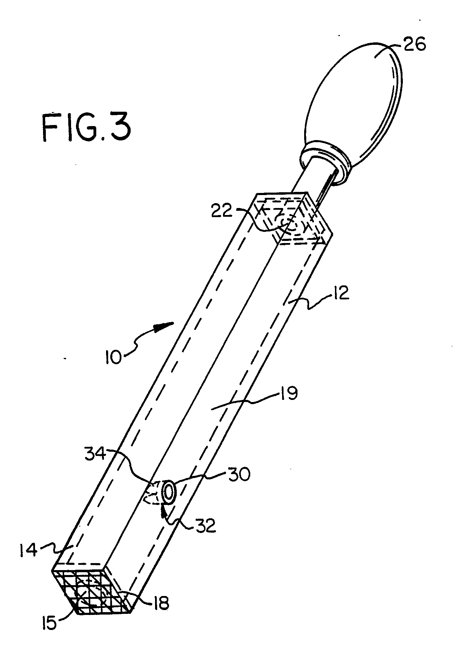

The device illustrated in the figures may be operated by initially evacuating the hollow member 12. For the embodiment of Figures 1 and 3, this involves first squeezing or compressing the aspirator bulb 26. The device illustrated in Figure 2 may be operated by first pressing on the extended end of the shaft 30 that is attached to the piston 28 which forces the plunger 28 into its lowermost position in contact with one or more stops (not shown) or a constriction at the lower end of the hollow member 12. While maintaining the bulb 26 (Figure 1) in a depressed or squeezed form or the plunger 28 (Figure 2) in the lowermost position, the active or lower end 14 of the hollow member 12 is placed in the liquid sample such that the opening 15 having the membrane 18 secured therein is placed below the surface of the liquid sample and the bulb 26 is released slowly (or the plunger 28 is withdrawn slowly) in order to create a partial vacuum in the device 10 and draw liquid through the membrane 18. Alternatively, the bulb 26 may be both squeezed and released (or the plunger 28 pressed to its lowermost position and withdrawn) while the opening 15 is beneath the surface of the liquid. As liquid passes through the membrane 18 and fills the reservoir 24 of the hollow member 12, insoluble solid matter present in the original liquid sample is collected on the outermost surface of the membrane 18. Either before or after removal of the active end of the hollow member 12 from the liquid sample, preferably before, pressure may be increased within the hollow member 12 by squeezing the bulb 26 or depressing the plunger 28, thereby causing liquid to flow from the reservoir through the porous membrane 18. Depending upon the particular test being performed, this cycle of drawing liquid into the reservoir 24 and expelling the liquid therefrom may be repeated several times. If the liquid sample suspected of containing the analyte contains a high level of particulate or gel-like, membrane blocking material, the last pressure cycle should end with rapid liquid expulsion from the hollow member 12, thereby purging the outer membrane surface of debris, leaving it clear for subsequent reagents and/or wash solutions and for observation of any color change. The use of such reagent and/or wash solution to treat the membrane surface may be conducted in the same manner as the sample solution suspected of containing the analyte is drawn into and expelled from the reservoir 24 through the membrane 18.

Use of the enhanced flow device is not limited to situations in which insoluble membrane blocking material, such as particulate or gel-like material, must be purged from the membrane surface. In certain diagnostic tests, it may be preferable to retain a particular material on the membrane surface. For example, it may be desirable to separate bacteria present in a urine sample to undergo testing. In such an instance, using an embodiment such as those illustrated in Figures 1 and 2, the pressure cycle may be terminated with reduced pressure within the hollow member 12. Thus, rather than expelling all the liquid from the reservoir 24 with the opening 15 positioned below the surface of the sample liquid, the bulb or plunger is released or withdrawn, respectively, as the lower end 14 of the device is removed from the liquid. This will serve to retain bacteria on the membrane surface. This is more easily accomplished with a device similar to that shown in Figure 2 than that of Figure 1 since a tight fitting plunger, perhaps provided with one or more sealing rings, such as O-rings 29, is more effective in maintaining a reduced pressure within the reservoir than the embodiment illustrated in Figure 1. Once the debris is retained on the surface of the membrane, additional pressure cycles are not typically performed and the device is handled like a conventional dipstick.

Figure 3 illustrates a further embodiment which permits interfering solid matter to be trapped within the hollow member 12, particularly on the inner face of the porous structure 18 facing the reservoir 24. This device includes, in addition to the first or lower opening 15 and the second or upper opening 22, an additional or third opening 30. The additional opening 30 has located therein a valve means, particularly a one-way or check valve 32. The opening 30 and check valve 32 are located, preferably, closer to the first opening 15 than the second opening 22.

Most preferably, the first opening including the membrane 18 and the third opening including the check valve 32 are located proximate one another. The check valve 32 is so devised as to permit ingress of liquid into the elongate member through the third opening 30 while retarding egress of liquid from the elongate member through the third opening. The check valve 32 preferably employs two flexible members 34 which are biased toward each other to allow ingress of fluid into the elongate hollow member 12 when a reduced pressure, that is, a partial vacuum, is created in the reservoir 24 of the hollow elongate member. The flexible members assume a closed position when the pressure within the hollow elongate member 12 is substantially at or exceeds atmospheric pressure.

In operation, the device 10 is placed in a liquid sample such that both the first opening 15 including the membrane 18 and the third opening 30 including the check valve 32 are positioned below the surface of the liquid. The means for creating a differential pressure associated with the second opening 22 is actuated, by compressing the bulb 26 (or pressing the plunger 28 into the passage of the elongate member) either before or after placing the device into the liquid. Once the membrane and check valve are below the surface of the liquid, the bulb 26 is released (or the plunger 28 is withdrawn) to create a partial vacuum within the reservoir 24. This causes the flexible members 34 to separate and liquid to flow into the reservoir. When the pressure in the interior of the hollow elongate member 12 is substantially equal to that on the exterior thereof, fluid flow into the elongate member 12 ceases and the check valve 32 closes. Under the effect of a positive pressure differential between the interior of the elongate member 12 and the exterior environment, produced by again compressing the bulb 26 (or forcing the plunger 28 into the hollow elongate member), liquid passes through the membrane 18. As in the other embodiments described above, the membrane is fully exposed to and in contact with the liquid samples being tested because flow is being directed through the membrane when a full cycle is performed. Several cycles of liquid sample inspiration and expulsion may be easily achieved with this embodiment.

Several advantages accrue from the use of this embodiment as compared to conventional test strips, and even with respect to the embodiments of Figures 1 and 2, particularly when test samples are being analyzed having high concentrations of insoluble solid material. With conventional devices, the abundance of soluble matter tends to cling to the test surface, hinder diffusion, and obscure the testing performance. With the embodiments illustrated in Figures 1 and 2, although generally effective in purging insoluble material from the outer face of the membrane 18, lower flow rates and more difficulty in filling the reservoir 24 may be encountered as compared to the device illustrated in Figure 3, since the insoluble material begins to form a cake on the outer face of the membrane, thereby blocking the pores of the membrane as the reservoir is being filled with liquid. This resistance to flow with liquid samples containing high proportions of insoluble material may occur both because of the blockage of the pores of the membrane and because the maximum pressure differential which can be generated under ideal conditions on the intake or filling step is one atmosphere. In the embodiment illustrated in Figure 3, the reservoir 24 is filled during the inspiration or intake step by liquid flowing through the intake valve where little or no resistance occurs. Formation of a cake of insoluble material occurs on the inner face of the membrane during the expulsion step when liquid is being forced through the membrane. However, during the expulsion step, pressure differentials greatly exceeding one atmosphere can be achieved. Thus, although resistance to flow does occur as a cake of insoluble material accumulates on the inner face of the membrane, the larger pressure differentials generated during the expulsion step permit rapid expulsion of liquid from the device.

A further advantage to the embodiment illustrated in Figure 3 is that all insoluble material which might obscure the results of a test performed on the membrane, is trapped on the face of the membrane facing the interior of the reservoir. The outer face of the membrane, which is normally viewed by the user, does not, therefore, collect insoluble material.

As a result, color changes which occur on the surface of the membrane due to the interaction of a test reagent and an analyte may be easily seen on the outer face of the membrane. Even in those instances in which the analyte is insoluble (as, for example, a bacteria) but also undergoes a color change when in the presence of a test reagent, the outer face of the membrane may be examined for evidence of a color change since many of the membranes which are suitable become translucent when wet.

As indicated above, devices are preferred, at least in part, which have square or rectangular configurations or cross-sections because of the ease of manufacture of such devices. When embodiments are assembled in which the porous structure 18 is located at an opening at the bottom or tip of the elongate member, preferably an embodiment in which the side walls define the bottom opening, assembly may be accomplished rapidly and a high volume of units produced. With the manufacture of such an embodiment, the elongated tubes are secured in a holder with the open ends to which the porous structure is to be secured arranged facing upward and in the same plane. Preferably, the elongate members are arranged in close proximity or in contact with one another in a square or rectangular grid arrangement, although a single row of units may be employed. In the rectangular grid arrangement, a sheet of membrane large enough to cover all the open ends of the elongate members (or a strip of membrane when the arrangement includes a row of elongate members) is placed over the openings in the ends of the elongate members. When the elongate members are formed from a plastic material, a heating means, such as a welding horn or ultrasonic thermal device may be used to bond the membrane to the elongate member at the periphery of the opening. Preferably, the elongate member has a rib or collar formed at the periphery of the opening to which the membrane is affixed which allows the membrane and the plastic material to fuse simultaneously. If the elongate members are formed from glass, an adhesive normally will be employed to bond the membrane to the elongate member.

After the membrane is bonded to the elongate members at the opening 15, in the method of manufacture employing a rectangular grid of elongate members, parallel knives are lowered simultaneously between the parallel columns of elongate members and then either the grid rotated 90° and the same knives brought down between the rows of elongate members or a second set of knives, arranged at right angles to the first set, are lowered to sever the membrane. Where a single row of elongate members is being produced, a set of parallel knives may be used to sever the membrane or a single knife may be used as the row of elongate members is advanced in order to sever the membrane one unit at a time. It is preferred that the ends to which the membrane is affixed be at least slightly tapered or have a beveled or chamfered surface where the membrane is affixed to the elongate member. This permits the knives to be lowered between the ends of the elongate members without contact therewith.

In the embodiment in which the membrane is located at a circular tip, the membrane of suitable size and shape, preferably slightly oversized, is typically produced with a punch and the individual membrane disc initially placed on and tacked by a heating method to the periphery of the opening, followed by a second heat treatment to bond the oversized portion or flap of the membrane material to the elongate member.

When the membrane is located at an opening placed in a side wall of the elongate member, a group of elongate members is arranged adjacent and/or in contact with one another, all openings facing upward in the same plane. A strip of membrane material is placed over the openings and affixed, by thermal or adhesive means, to the periphery of each opening, in a manner similar to that described above. One or more knives, arranged parallel to one another, may be employed to sever the membrane in a manner also similar to that described above.

## Claims

1. A flow enhanced immunodiagnostic device comprising:
a hollow elongate member having first and second openings; and
a porous liquophilic membrane positioned across said first opening, said porous liquophilic membrane containing an activating agent capable of reacting with a test reagent, said second opening adapted to be operatively associated with means for generating a pressure differential between the interior of said elongate member and the exterior thereof, whereby a liquid test sample may be introduced to the interior of said elongate member bypassing through said porous liquophilic membrane and any insoluble substances present in said liquid test sample accumulates on a surface of said porous liquophilic membrane.

2. A flow enhanced immunodiagnostic device according to claim 1 wherein said activating agent is covalently bound to said porous liquophilic membrane.

3. A flow enhanced immunodiagnostic device according to claim 1 wherein said porous membrane comprises a hydrophilic, microporous, skinless, polyamide membrane chemically bound to a residue of trichloro-s-triazine.

4. A flow enhanced immunodiagnostic device comprising:
a hollow elongate member having at least first and second openings;
a porous liquophilic membrane positioned across said first opening and containing an activating agent capable of reacting with a test reagent; and
means associated with said second opening for permitting ingress of liquid into said elongate member through said second opening while retarding egress of liquid from said elongate member through said second opening, whereby a liquid test sample maybe introduced to the interior of said elongate member through said ingress means and liquid is expelled from said reservoir exclusively through said porous liquophilic membrane while any insoluble substance present in said liquid test sample accumulates on an inner surface of said porous liquophilic membrane.

5. A flow enhanced immunodiagnostic device according to claim 4 wherein said hollow elongate member has a third opening adapted to be operatively associated with means for generating a pressure differential between the interior of said elongate member and the exterior thereof.

6. A flow enhanced immunodiagnostic device comprising:
a hollow elongate member having first and second openings; and
a porous liquophilic membrane positioned across said first opening, said second opening adapted to be operatively associated with means for generating a pressure differential between the interior of said elongate member and the exterior thereof, whereby a liquid test sample may be introduced into the interior of said elongate member bypassing through said porous hydrophilic membrane and any insoluble substances present in said liquid test sample accumulates on a surface of said porous liquophilic membrane.

7. A flow enhanced immunodiagnostic device comprising:
a hollow elongate member having at least first and second openings;
a porous liquophilic membrane positioned across said first opening; and
means associated with said second opening for permitting ingress of liquid into said elongate member through said second opening while retarding egress of liquid from said elongate member through said second opening, whereby a liquid test sample maybe introduced to the interior of said elongate member through said ingress means and liquid is expelled from said reservoir exclusively through said porous liquophilic membrane while any insoluble substance present in said liquid test sample accumulates on an inner surface of said porous liquophilic membrane.

8. A flow enhanced immunodiagnostic device according to claim 7 wherein said hollow elongate member has a third opening and means associated with said third opening for generating a pressure differential between the interior of said elongate member and the exterior thereof.

9. A flow enhanced immunodiagnostic device comprising:
a hollow elongate member having first and second openings; and
a porous liquophilic membrane positioned across said first opening, said porous liquophilic membrane containing a test reagent capable of reacting with an analyte and producing an indicia of the presence of the analyte, said second opening adapted to be operatively associated with means for generating a pressure differential between the interior of said elongate member and the exterior thereof, whereby a liquid test sample containing a substantially soluble analyte may be introduced into the interior of said elongate member by passing through said first opening and said porous liquophilic membrane and any insoluble substances present in said liquid test sample accumulates on a surface of said porous liquophilic membrane.

10. A flow enhanced immunodiagnostic device according to claim 9 wherein said porous membrane comprises a hydrophilic, microporous, skinless, polyamide membrane chemically bound to a residue of an activating agent, which residue is also bound to a biologically active material.

11. A flow enhanced immunodiagnostic device according to claim 9 wherein said means for generating a pressure differential comprises a piston having a shape commensurate with the internal configuration of the hollow elongate member.

12. A flow enhanced immunodiagnostic device according to claim 9 wherein said means for generating a pressure differential comprises an elastomeric bulb.

13. A flow enhanced immunodiagnostic device comprising:
a hollow elongate member having at least first and second openings;
a porous liquophilic membrane containing a test reagent capable of reacting with a predetermined analyte and producing an indicia of the presence of the analyte, said liquophilic membrane positioned across said first opening; and
means associated with said second opening for permitting ingress of liquid into said elongate member through said second opening while retarding egress of liquid from said elongate member through said second opening, whereby a liquid test sample containing an analyte may be introduced to the interior of said elongate member through said ingress means and liquid is expelled from said elongate member exclusively through said porous liquophilic membrane and said first opening while any insoluble substance present in said liquid test sample accumulates on an inner surface of said porous liquophilic membrane.

14. A flow enhanced immunodiagnostic device according to claim 13 wherein said hollow elongate member has a third opening and means associated with said third opening for generating a pressure differential between the interior of said elongate member and the exterior thereof.

15. A flow enhanced immunodiagnostic device according to any of claims 1, 4, 6, 7, 9, or 13 wherein the porous liquophilic membrane is a microporous hydrophilic membrane.

16. A flow enhanced immunodiagnostic device according to any of claims 9 or 13 wherein said test reagent comprises a biologically active material.

17. A flow enhanced immunodiagnostic device according to any of claims 1, 4, 6, 7, 9, or 13 wherein said porous membrane comprises a polyamide membrane.

# FIG. I

FIG.2

# FIG.3